# EUROPEAN PATENT SPECIFICATION

(11) **EP 4 030 982 B1**
(45) Date of publication and mention of the grant of the patent: **07.06.2023**
(21) Application number: 20788992.4
(22) Date of filing: 18.09.2020
(51) Int. Cl.: A61B 1/00, A61B 1/267

(54) **ENDOSCOPE**
ENDOSKOP
ENDOSCOPE

(30) Priority: 20.09.2019 GB 201913613
(43) Date of publication of application: 27.07.2022
(73) Proprietor: Intersurgical AG, 9490 Vaduz (LI)
(72) Inventor: MILLER, Andrew, Wokingham Berkshire RG41 2RZ (GB); RUEDI, Rene Werner, Wokingham Berkshire RG41 2RZ (GB)
(74) Representative: Adamson Jones
(86) International application number: PCT/EP2020/076171
(87) International publication number: WO 2021/053181

(56) References cited:
- WO-A2-2010/065566
- US-A1- 2014 275 772
- US-A1- 2018 207 403

## Description

The present invention relates to an endoscope, and more particularly to a flexible endoscope for aiding intubation.

Endoscopes are devices for examining the interior of a hollow organ or cavity of the body. Endoscopes typically comprise a handle, an operative member, which may be rigid or flexible, and means for transmitting an image from the distal end of the operative member, situated within a patient's body during use, to an eyepiece or display outside the body. Examples of flexible endoscopes include fiberscopes and videoscopes. Fiberscopes typically comprise a bundle of optical fibres within the operative member for transmitting an image from a lens to an eyepiece. As a result of the optical fibres, in particular, fiberscopes are both fragile and expensive. Videoscopes include a camera in the operative member and a connected video display. Videoscopes require a longer setup time, and take up more space.

Recently, endoscopes have also been specifically configured to aid intubation. Intubation is the process of inserting an endotracheal tube through the mouth and into the trachea of a patient. This allows the patient to be connected to a ventilator to assist with respiration. This may be because the patient is unable to breathe without assistance, for example during anaesthesia, or because the patient is unable to maintain their airway.

A stylet is an example of a conventional device for aiding intubation. A stylet is a malleable device that is inserted into an endotracheal tube prior to insertion of the endotracheal tube into the trachea of a patient. The malleable nature of the stylet allows a user to pre-form the stylet, which then maintains the surrounding endotracheal tube in a desired shape prior to insertion into the trachea. The endotracheal tube is then introduced into the trachea using a laryngoscope, such that a distal end of the endotracheal tube is located within the trachea, and a proximal end of the endotracheal tube projects from the patient's mouth. Once the distal end of the endotracheal tube is appropriately positioned within the trachea, the stylet is withdrawn, along with the laryngoscope.

A bougie is another example of a conventional device for aiding intubation. A bougie is a flexible device that is used to provide a positive position within the trachea for guiding an endotracheal tube into the trachea. A bougie is typically introduced into the trachea such that a distal end is located within the trachea and a proximal end projects from the patient's mouth. An endotracheal tube is then "railroaded" over the bougie, which involves inserting the proximal end of the bougie into a distal end of the endotracheal tube, and sliding the endotracheal tube over, and along, the external surface of the bougie until the distal end of the endotracheal tube is appropriately positioned within the trachea. The bougie therefore acts as a guide for placement of the endotracheal tube. The bougie is then removed from the endotracheal tube and the patient.

Endoscopes enable the user to see images within the body, eg near the tip of the endotracheal tube whilst inserting an endotracheal tube into the trachea, for example by those methods described above or otherwise. Flexible endoscopes generally work in one of two ways, dependent on their length. Longer endoscopes are used to pre-load an endotracheal tube which is retained at the proximal end of the operative member, and then advanced over the operative member once the distal end of the operative member is correctly located within the trachea of a patient. Shorter endoscopes are used only to visualise the vocal chords of the patient whilst an endotracheal tube is introduced into the trachea, so that the placement of the endotracheal tube can be guided and adjusted accordingly.

WO2010/065566 discloses an intubating stylet that has a proximal end and a distal end. The intubating stylet includes a housing at the proximal end of the stylet and a tip portion at the distal end of the stylet. The tip portion has a distal gear at its proximal end. An outer shaft extends from the housing to the tip portion and contains an inner shaft defining a hollow lumen. The stylet includes a drive member in the housing and a control member coupled with the drive member. The drive member is coupled to the inner shaft and configured to rotate the inner shaft, and the control member is configured to receive a user input and to translate the input into operation of the drive member. US2018/207403 discloses a tracheoscope that includes a handle having a control mechanism, where one end of the handle is connected with a plug-in type screen, and the other end of the handle is combined with a catheter. US2014/275772 discloses a one piece, one step, steerable video intubation device that has an intubation device body having a downwardly extending stylet, a proximal handle, and a video display.

There has now been devised an improved endoscope, which overcomes or substantially mitigates disadvantages associated with the prior art.

According to a first aspect of the invention, there is provided an endoscope for use in intubation, the endoscope comprising a housing, a display device having a display screen, and an operative member extending from the housing, the operative member being configured to be inserted into a patient's airway and to transmit images to the display screen, the housing comprising an actuator coupled to the operative member and a retainer coupled to the display screen, the housing having a handle portion comprising at least the actuator, and the actuator being single-handedly rotatable by an operator relative to the retainer, such that the operative member is rotatable relative to the display screen, wherein the actuator and the retainer are rotatable relative to each other about a longitudinal axis of the operative member and/or a longitudinal axis of the handle portion.

The present invention is advantageous principally because the housing of the endoscope comprises an actuator coupled to the operative member and a retainer coupled to the display screen, and the actuator is single-handedly rotatable by an operator relative to the retainer, such that the operative member is rotatable relative to the display screen. This enables an endoscope to have a display screen that is mounted to, or integral with, the housing of the endoscope, without the display screen rotating relative to the operator when the operative member is rotated.

This is in contrast to a display screen mounted on to an endoscope known in the art, since those endoscopes are rotated by a user rotating the housing with their hand, with which the mounted display would also rotate.

According to a further aspect of the present invention there is provided a method of manufacturing an endoscope as defined above, the method comprising the steps of:
(i) providing a housing, a display device having a display screen, and an operative member extending from the housing, the operative member being configured to be inserted into a patient's airway and to transmit images to the display screen, and the housing comprising an actuator coupled to the operative member and a retainer coupled to the display screen, and
(ii) arranging the housing to have a handle portion comprising at least the actuator, and the actuator to be single-handedly rotatable by an operator relative to the retainer, such that the operative member is rotatable relative to the display screen, wherein the actuator and the retainer are rotatable relative to each other about a longitudinal axis of the operative member and/or a longitudinal axis of the handle portion.

The actuator and the retainer may be arranged such that they are engageable by first and second parts of the operator's body, the first and second parts of the operator's body being capable of movement relative to each other. The first part of the operator's body may be a part of a first hand and associated fingers and thumb of the operator. The second part of the operator's body may be a different part of the first hand and associated fingers and thumb of the operator. Alternatively, the second part of the operator's body may be another part of the body of the operator.

The handle portion may comprise the actuator and the retainer. That is, the actuator and the retainer may be parts of the handle portion. In this arrangement, the actuator may be arranged such that it is engageable by at least one finger or thumb of the operator, and the retainer may be arranged such that it is engageable by at least one different finger or thumb of the same hand of the operator. The actuator may be movable through at least a first movement path. The first movement path may be an arcuate movement path. The operator may be able to effect rotation of the actuator and the retainer relative to each other through relative movement of the fingers of a first hand, or through the relative movement of a finger and the thumb of the first hand. For example, the actuator may be positioned towards the top end of the handle portion when held by the operator in use, such that the actuator is arranged to be engageable by the first finger of the operator, ie the finger closest to the operator's thumb, or the thumb of the operator, and the retainer may be the remainder of the handle portion, such that the retainer is engageable by the remainder of the operator's hand. Thus, when the operator moves their first finger or thumb relative to the rest of their hand, the actuator will rotate relative to the retainer, causing rotation of the operative member whilst maintaining the position of the display screen relative to the operator.

Alternatively, the handle portion may comprise the actuator, but not the retainer. That is, the retainer may be positioned remote from the handle portion. In this arrangement, the actuator may be arranged to be engaged by a hand and associated fingers of the operator, and the retainer may be arranged to be engaged by a different part of the operator's body. The operator may be able to effect rotation of the actuator relative to the retainer through movement of their hand relative to the different part of their body. For example, the retainer may extend from the handle portion so as to engage with, eg rest upon, the operator's wrist or arm, the wrist or arm being for the hand engaging the actuator. Thus, when the operator moves their fingers or hand relative to their wrist or arm, the actuator will be rotated relative to the retainer, causing rotation of the operative member whilst maintaining the position of the display screen relative to the operator.

The operative member may extend from the housing substantially parallel to a longitudinal axis of the handle portion.

The axis of rotation may be generally or substantially aligned with a central longitudinal axis of the operative member and/or with a central longitudinal axis of the handle portion.

Generally or substantially aligned with the central longitudinal axis of the operative member or with the central longitudinal axis of the handle portion may be defined as being generally or substantially parallel to the central longitudinal axis of the operative member or to the central longitudinal axis of the handle portion respectively, for example at an angle of less than 45 degrees, 30 degrees, 15 degrees, or 5 degrees, to the central longitudinal axis.

Generally or substantially aligned with the central longitudinal axis of the operative member or with the central longitudinal axis of the handle portion may be defined as being positioned generally or substantially centrally in a cross-section through the operative member or the handle portion, for example within 50%, 25%, 10% or 5% of the radius of the cross-section of the operative member or the handle portion, where the cross-section through the operative member or the handle portion is perpendicular to the central longitudinal axis of the operative member or the handle portion.

The operative member may be rotatable relative to at least part of the handle portion of the housing, eg relative to the part of the handle portion upon which the palm of the operator's hand is located. The actuator may comprise a first actuator member for controlling rotation of the operative member relative to the handle portion, ie about a longitudinal axis of the handle portion and/or the operative member.

This is advantageous in that the operative member may be controlled without having to move or rotate at least part of the handle portion of the housing, eg that part of the handle portion upon which the palm of the operator's hand is located, which provides improved control during use. This is particularly beneficial since in use the endoscope is held high, eg above the head of the operator, in order to introduce the operative member into the airway of a patient, making rotation of the part of the handle portion upon which the palm of the operator's hand is located difficult.

The first actuator member may be mechanical in form. Where the first actuator member is mechanical in form, the first actuator member may include a moveable member, which may be operated by a user's finger, for example. The first actuator may be arranged at the opposite end of the housing relative to the end from which the operative member extends. The first actuator member may be arranged for pivoting or rotational movement. The first actuator member may be pivotable or rotatable relative to the handle portion, for example about a longitudinal axis of the operative member and/or the handle portion. The first actuator member may have the form of an arm. Alternatively, the first actuator member may comprise a wheel, the wheel being rotatable within the housing, for example about a longitudinal axis of the operative member and/or the handle portion. The wheel may be positioned within a slot in the housing. The wheel may be arranged at the opposite end of the housing relative to the end from which the operative member extends. The wheel may be mechanically coupled to the operative member such that rotation of the wheel causes rotation of the operative member. The wheel may be rotatable in a first direction to rotate the operative member in a first direction relative to the handle portion. The wheel may be rotatable in a second direction to rotate the operative member in a second direction relative to the handle portion. The first and second directions may be rotationally opposite directions. The wheel may be operable by a user's finger.

Alternatively, the actuator may be formed by a head portion of the housing, which is rotatable relative to the remainder of the handle portion and the display screen device. The handle portion and the display screen device may be fixed relative to each other. The rotatable head portion may be arranged at the opposite end of the housing relative to the end from which the operative member extends. The rotatable head portion may be arranged relative to the handle portion of the housing such that it may be rotated by the index finger and/or thumb of the user.

A distal tip portion of the operative member may be movable relative to a main body of the operative member, and this movement of the distal tip portion of the operative member may be controllable by the user from the housing of the endoscope, for example from the actuator. This controlled movement of the distal tip portion of the operative member facilitates both the viewing of the interior of the patient's body and the guiding of the endotracheal tube during intubation.

The movement of the distal tip portion may comprise variation of the angle of orientation of the distal tip portion relative to the main body portion of the operative member, eg about a hinge or pivot portion, or about a bending portion. The distal tip portion may therefore be angled, ie non-parallel, relative to a main body portion of the operative member, and may for example, be obliquely angled relative to the main body portion. Alternatively, the distal tip portion may be angled as much as 180 degrees relative to the main body portion of the operative member. The shape of the distal tip portion may remain substantially constant during this movement.

The housing may comprise a second actuator member for controlling movement of the distal tip portion of the operative member, ie to vary the angle of orientation of the distal tip portion of the operative member relative to the main body of the operative member, eg within the plane in which the central longitudinal axes of the operative member and the housing lie, which may be the median plane of the patient's body, in use. The second actuator member may be mounted on the actuator of the housing. In preferred embodiments, the second actuator member may be mounted on the first actuator member, or vice versa.

The first actuator member and the second actuator member may be arranged to be single-handedly operated by an operator, eg using the same or different fingers of the same hand. The first actuator member and the second actuator member may each be operated using a single digit of the same hand of the operator. The first actuation member may be operable by a first single digit, whilst the second actuation member is operable by a second single digit. The first actuation member may be operable by a first single digit, whilst the second actuation member is simultaneously operable by a second single digit. The first digit and the second digit may be the same or different digits of the same hand of an operator.

The second actuator member, herein referred to as a distal tip portion actuator, may be mechanical in form. The distal tip portion actuator may have an engagement portion, the engagement portion being operable by a user. Where the distal tip portion actuator is mechanical in form, the engagement portion may be a moveable arm, which may be operated by a user's thumb, for example. The engagement portion may be movable through at least a second movement path. The second movement path may reside substantially in a single plane. The second movement path may be substantially arcuate. In this arrangement, the distal tip portion actuator may be moveable by a user relative to a longitudinal axis of the housing, eg either linearly or by rotation through an arc. For example, the engagement portion may comprise a lever projecting from a slot in the housing, within which the lever is movable relative to a longitudinal axis of the housing. Where the engagement portion is a lever, the second movement path is preferably arcuate. The distal tip portion actuator may be moveable relative to a longitudinal axis of the housing in a first actuator member direction to move and/or vary the orientation of the distal tip portion in a first operative direction, and the distal tip portion actuator may be moveable relative to a longitudinal axis of the housing in a second actuator direction to move and/or vary the orientation of the distal tip portion in a second operative direction. The first and second actuator directions may be opposite directions, either rotationally through an arc or linearly, and the first and second operative directions may be opposite directions, either rotationally through an arc or linearly.

Alternatively, the distal tip portion actuator may comprise a pair of buttons, which may be depressed by a user's thumb or a pair of fingers, for example. The engagement portion of a first button may be movable through at least a first movement path, and the engagement portion of a second button may be movable through at least a second movement path. The first and second movement paths may reside substantially in a single plane. The first and second movement paths may be substantially linear. In this arrangement, each of the buttons may be moveable or pressable by a user substantially perpendicularly to a longitudinal axis of the housing. The first button may be moveable or pressable by a user to move and/or vary the orientation of the distal tip portion in a first operative direction. The second button may be moveable or pressable by a user to move and/or vary the orientation of the distal tip portion in a second operative direction.

The distal tip portion actuator may be mounted on the first actuator member. For example, where the first actuator member is rotatable within or relative to the housing, the distal tip portion actuator may be moveable or pressable relative to the first actuator member. In a preferred embodiment, the first actuator member may be rotatable about a longitudinal axis of the housing, and the distal tip portion actuator member may be movable at least partially in a direction that is substantially parallel to the longitudinal axis of the housing. Alternatively, the first actuator member may be rotatable about a longitudinal axis of the housing, and the distal tip portion actuator member may comprise two or more buttons spaced apart on the first actuator member, for example arranged along a longitudinal axis of the housing. The two or more buttons may be pressable substantially perpendicularly to a longitudinal axis of the housing.

Alternatively, the engagement portion of the distal tip portion actuator may be a wheel, the wheel being rotatable within the housing, for example about a longitudinal axis of the operative member and/or the handle portion. The wheel may be positioned within a slot in the housing. The wheel may be arranged at the opposite end of the housing relative to the end from which the operative member extends. The wheel may be mechanically coupled to the operative member. The wheel may be rotatable about a longitudinal axis of the housing in a first actuator direction to move and/or vary the orientation of the distal tip portion in a first operative direction, and the wheel may be rotatable about a longitudinal axis of the housing in a second actuator direction to move and/or vary the orientation of the distal tip portion in a second operative direction. The first and second actuator directions may be rotationally opposite directions. The wheel may be operable by a user's finger.

This is advantageous in that it allows an operator to control movement of the operative member single-handedly.

The second actuator member, eg the distal tip portion actuator, may be disposed on a first surface of the housing, the first surface being on the same side of the housing as the display screen. Where the housing includes a head portion arranged at the opposite end of the housing relative to the end of the housing from which the operative member extends, a second actuator member, eg the distal tip portion actuator, may be disposed on a first surface of the head portion of the housing, the first surface being on the same side of the housing as the display screen, such that the distal tip portion actuator may be operated by the user's thumb.

The provision of a first actuator member formed on a portion of the housing, eg a head portion of the housing, and that portion including the second actuator member, eg a distal tip portion actuator, allows movement of the operative member to be controlled single-handedly.

The first actuator member described above may be disposed on a second surface of the housing, for example a second surface of the head portion of the housing, the second surface being on the opposite surface of the housing as the distal tip portion actuator and the display screen, such that the first actuator member may be operated by a user's first finger.

This combination of features may be particularly beneficial in terms of the usability of the device, and hence, according to a further aspect of the invention, there is provided an endoscope for use in intubation, the endoscope comprising a housing including a handle portion, a display device having a display screen, an operative member extending from the housing, the operative member being configured to be inserted into a patient's airway and to transmit images to the display screen, a first actuator member disposed on a first surface of the handle portion and coupled to the operative member, and a second actuator member disposed on a second surface of the handle portion and coupled to the operative member, the first and second surfaces being opposite surfaces of the handle portion.

The first actuator member may be arranged to control rotational movement of the operative member relative to any or all of the housing, the display screen and the handle portion. The second actuator member may be arranged to control movement of the distal tip portion of the operative member, ie to vary the angle of orientation of the distal tip portion of the operative member relative to the main body of the operative member, eg within the plane in which the central longitudinal axes of the operative member and the housing lie.

The first actuator member and the second actuator member may be arranged to be single-handedly operated by an operator, eg using different fingers of the same hand. The first actuator member and the second actuator member may each be operated using a single digit of the same hand of the operator. The first actuation member may be operable by a first single digit, whilst the second actuation member is operable by a second single digit. The first actuation member may be operable by a first single digit, eg a finger, whilst the second actuation member is simultaneously operable by a second single digit, eg a thumb. The first digit and the second digit may be different digits of the same hand of an operator.

In an alternative arrangement, the housing may comprise a single actuator for controlling rotation of the operative member relative to the handle portion and for controlling movement (ie articulation) of the distal tip portion of the operative member. The single actuator may be movable through at least two paths. The two paths may each extend between two end points. The two paths may be different paths. The two paths may be different in that they each have two differing end points, ie the two paths do not share any end points. The single actuator may be movable through at least a first movement path to control rotation of the operative member relative to the handle portion. The first movement path may be substantially arcuate, for example. The single actuator may be movable through at least a second movement path to control articulation of the distal tip portion of the operative member. The second movement path may be substantially linear, for example. The single actuator may be movable through the first movement path to control rotation of the operative member relative to the handle portion, and at any point along that first movement path may be moveable along at least one further movement path to control articulation of the distal tip portion of the operative member.

The single actuator may comprise an engagement portion, engageable by an operator. The engagement portion may protrude from the housing, such that it is engageable by at least one finger or thumb of the operator. The engagement portion may include a movable member. The movable member may be arranged for linear and/or pivoting and/or rotational movement. The movable member may have the form of an arm. The movable member may be movable in all directions within a geometric surface, within boundaries defined by the actuator, for example about a ball joint. That is, the geometric surface may be a curved geometric surface. The single actuator may be a joystick, for example.

The housing may further comprise a grip portion, for engagement by at least the index finger of the user. The grip portion may be disposed on a second surface of the housing, the second surface being on the opposite side of the housing to the display screen. Where the housing includes a head portion arranged at the opposite end of the housing relative to the end of the housing from which the operative member extends, the grip portion may be disposed on a second surface of the head portion of the housing, the second surface being on the opposite side of the housing to the display screen, such that the grip portion may be engaged by at least the user's index finger.

According to a further aspect of the invention, there is provided an endoscope for use in intubation, the endoscope comprising a housing including a handle portion and a display screen portion, the display screen portion having a display screen, and an operative member extending from the housing, the operative member being configured to be inserted into a patient's airway and to transmit images to the display device, and an arm portion that extends between the handle portion of the housing and the display screen portion of the housing , such that the display screen is spatially separated from the handle portion.

The arm portion extending between the handle portion of the housing and the display screen portion of the housing portion, such that the display screen is spatially separated from the handle portion, is advantageous in that it enables an arrangement in which the display screen is easily visible to an operator in use, regardless of whether the endoscope is at the commencement of insertion into the patient's airway, eg when the operator's arm is fully raised, or the endoscope is fully inserted into the patient's airway, eg when the operator's arm is lower.

The arm portion extending between the handle portion of the housing and the display screen portion of the housing portion, such that the display screen is spatially separated from the handle portion, also enables an arrangement in which a user's wrist is positionable between the display screen portion and the handle portion of the housing. This is advantageous in that the positioning of the display screen ensures that it is not obscured by the operator's wrist in use.

According to a further aspect of the present invention there is provided a method of manufacturing an endoscope as defined above, the method comprising: providing a housing including a handle portion and a display screen portion, the display screen portion having a display screen, an operative member extending from the housing, the operative member being configured to be inserted into a patient's airway and to transmit images to the display device, and an arm portion that extends between the handle portion of the housing and the display screen portion of the housing, such that the display screen is spatially separated from the handle portion.

The display screen portion may include the window through which the display screen is viewable. The display screen portion may have a generally cuboidal portion within which the display device is accommodated. The display screen may therefore be spatially separated from the handle portion of the housing, eg such that the user's wrist is positionable between the display screen portion and the handle portion of the housing.

The display screen portion may be disposed proximal to the end of the housing from which the operative member extends. The arm portion may extend towards the user, when in use, such that the display screen portion is closer to the user than the handle portion, in use. The arm portion may extend from the end of the housing from which the operative member extends.

The handle portion may be substantially cylindrical. The handle portion may be of a length and/or circumference similar to that of the average grip size of a human hand. The handle portion may have a central longitudinal axis that is curved.

The arm portion may be arranged to rest against the inside or outside of a user's wrist. The arm portion of the housing may have a cross-sectional width that is less than the cross-sectional width of the display screen portion of the housing. The arm portion may have a cross-sectional width that is less than the cross-sectional width of the handle portion. The arm portion may extend substantially perpendicularly from the handle portion, eg at an angle of 70-120° thereto. The display screen may be positioned at the opposite end of the arm portion relative to the handle portion, ie the distal end of the arm portion.

The separation between the operative surfaces of the display screen and the handle may be at least 1 cm, at least 2cm, at least 5cm or at least 10 cm.

The display screen may be an LCD screen. This is advantageous in that the display screen is inexpensive to produce, reducing the overall manufacture cost of the endoscope and allowing it to be implemented as a single-use endoscope. Alternatively, the display may be an OLED screen.

The central viewing axis of the display screen may be obliquely angled relative to the longitudinal axis of the arm portion, eg upwardly towards the eyes of the user, and the central viewing axis of the display screen may be obliquely angled relative to the central longitudinal axis of the handle portion. Specifically, the top edge of the display screen may be tilted towards the handle portion. The display screen may be substantially square or substantially rectangular in shape.

The operative member may extend from an end of the housing. The operative member may extend from the housing in a direction that is substantially perpendicular to the arm portion of the housing, eg at an angle of 70-120°. This is advantageous in that it enables that the position of the display screen to be above the operative member, in use, such that the display screen can be easily viewed regardless of whether the operative member is partially inserted into a patient's mouth or fully inserted into the patient's trachea.

The housing may further comprise a head portion. The head portion of the housing may be substantially cylindrical. The head portion of the housing may be of a similar diameter to the handle portion. The arm portion of the housing may extend from a first end of the handle portion. The head portion may extend from a second end of the handle portion. The first end and the second end may be opposite ends. The operative member may be substantially greater in length than it is in diameter. The operative member may be of a length at least substantially similar to the distance between the mouth and trachea of an adult or infant human. However, preferably the operative member is of a length substantially greater than the distance between the mouth and trachea of an adult or infant human, which allows an endotracheal tube to be loaded and then advanced over the endoscope once the distal end of the operative member is correctly located within the trachea of a patient The operative member may be of a diameter substantially less than the average diameter of a human's trachea. The operative member may be of a diameter substantially less than the average diameter of an endotracheal tube.

The operative member may be configured to be received within an endotracheal tube. The operative member may be shaped and/or dimensioned to be received within an endotracheal tube. The operative member may be substantially elongate in form. The operative member may have a diameter, for example an outer diameter, of between 1.0mm and 10.0mm. The operative member may have a diameter of between 2.0mm and 5.0mm. The operative member may have a length between 100mm and 1000 mm. The operative member may have a length between 200mm and 700mm.

The distal end of the operative member may comprise a rounded tip. By distal end is meant an end of the operative member that enters the trachea of a patient first during use. The rounded tip may comprise a circular cross-section and/or may comprise a substantially hemi-spherical global form.

The operative member may comprise a light source for illuminating an interior surface of the body, from which the images transmitted to the display device are obtained. The light source may be positioned at the distal tip of the operative member. The light source may be an LED.

Alternatively, the light source may comprise one or more light guides, eg optical fibres, that extend from a lamp in the housing to the distal end of the operative member. The lamp in the housing may be an LED. The operative member may be configured to receive light at its distal end, eg light from the light source that is reflected by the interior surface of the body being imaged, to form the images that are transmitted to the display device. The light may be received through an aperture and/or a lens at the distal end of the operative member.

The endoscope may comprise a device for converting the images into electronic format, eg an image sensor. The display device may receive the images in electronic format, eg a conventional image or video format. The image sensor may be adapted to capture still images and/or video sequence images.

The image sensor may be disposed at the distal end of the operative member. In this arrangement, the images may be transmitted in electronic format to the display device, for example by a wired connection though the operative member and the housing to the display device. Alternatively, the light forming the images may be transmitted to an image sensor accommodated by the housing, at the proximal end of the operative member. In this arrangement, the operative member may comprise one or more light guides, eg optical fibres, that extend from the distal end of the operative member to the image sensor in the housing.

The images may be instantaneous images. That is, in use, the display screen may display real-time images of the patient's trachea. The images may be single images. This is advantageous in that it allows a user to view adjustments to the positioning of the endotracheal tube within the trachea in real time. Alternatively, the images may be video images.

The operative member may comprise a channel therethrough. The channel may extend parallel to the longitudinal axis of the operative member. The channel may comprise an opening at proximal and distal ends of the operative member. Where the operative member comprises such a channel, the housing may comprise an aperture leading to the opening at the proximal end of the operative member, such that the channel is accessible through the housing. The channel may be used to insert or withdraw fluid from a patient's trachea in use.

According to a further aspect of the invention, there is provided an endoscope for use in intubation, the endoscope comprising a housing including a handle portion, a display device having a display screen, and an operative member extending from the housing, the operative member being configured to be inserted into a patient's airway and to transmit images to the display device, wherein the housing accommodates the display device, and the display screen is viewable through a window in the housing.

This aspect of the present invention is advantageous principally because the housing accommodates the display device, and the display screen is viewable through a window in the housing. This enables the endoscope, including the display device, to be provided as an integrated device, and hence enables the display device and the operative member of the endoscope to be packaged together in a sterile environment, eg as a single-use, disposable device, thereby reducing the risk of the spread of infection. On the contrary, video displays that are connected to videoscopes are used for multiple operations over a long period of time in order to justify their cost, and therefore require repeated sterilisation so as to reduce the risk of spread of infection.

The present invention also provides significant manufacturing advantages, such as reducing manufacturing costs.

The endoscope according to this aspect of the invention may therefore comprise a single-use, disposable device, which is provided in a single sterile package. The housing, including the display device accommodated therewithin, may be provided to the user as a single item, which does not require assembly by the end user. The housing may therefore be devoid of output connections to an external display device.

The display device may be accommodated within a display screen portion of the housing that is formed integrally with at least a handle portion of the housing. The display screen portion of the housing and/or the display device may be non-removable from the remainder of the housing of the endoscope, eg without disassembling or breaking the housing of the endoscope. The housing of the endoscope may comprise at least one housing component that defines a portion of the handle housing portion and the display screen housing portion.

The display screen housing portion may be formed from the same material as the handle portion. The display screen portion may be formed form the same material as the remainder of the housing.

The operative member may be connected to the housing of the endoscope via a connector. The operative member may be releasably attached to the connector, and therefore detachable from, and re-attachable to, the housing of the endoscope. Alternatively, the operative member may be permanently attached to the housing of the endoscope via the connector, ie not detachable, for example formed integrally therewith.

According to a further aspect of the present invention there is provided a method of manufacturing an endoscope as defined above, the method comprising the steps of:
(i) providing a housing including a handle portion, a display device having a display screen, and an operative member extending from the housing, the operative member being configured to be inserted into a patient's airway and to transmit images to the display device, and
(ii) assembling the housing to accommodate the display device, such that the display screen is viewable through a window in the housing.

Practicable embodiments of the invention will now be described, by way of example only, with reference to the accompanying drawings, of which:
Figure 1 is a front perspective view of an endoscope;
Figure 2 is a rear perspective view of the endoscope of Figure 1;
Figure 3 is an underside perspective view of the endoscope of Figures 1 and 2 connected to an operative member;
Figure 4 is a cross-sectional perspective view through the endoscope of Figures 1-3; and
Figure 5 is a rear perspective view of an endoscope similar to that of Figures 1-3, but having an alternative actuation mechanism for rotational movement of an operative member.

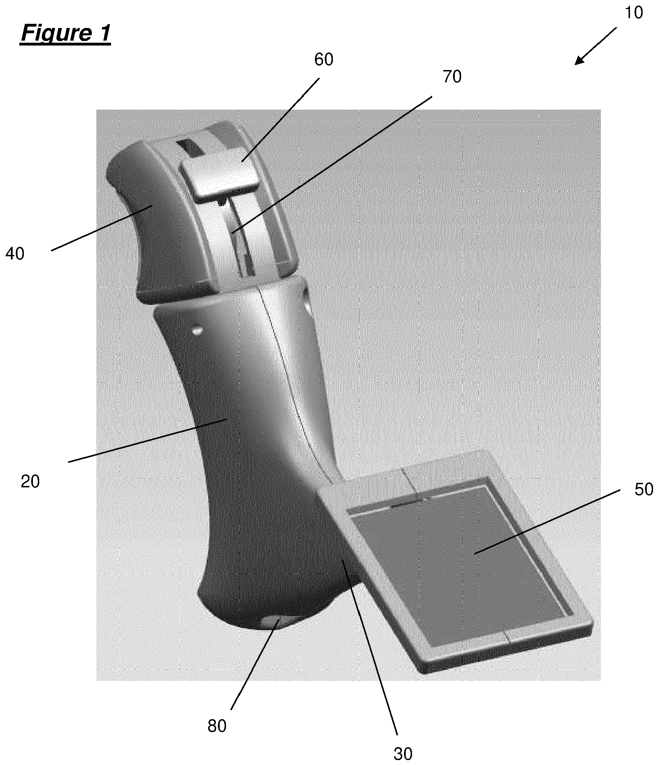

Figures 1 to 3 illustrate an endoscope 10 according to the present disclosure. The endoscope 10 comprises a body portion 20 having a head portion 40 at one end. Extending from the other end of the body portion 20 is an arm portion 30. At the other end of the arm portion 30 is an integrated display portion 50 comprising a display screen. The head portion 40 comprises an actuator 60 mounted within a slot 70. The body portion 20 further comprises a port 80 configured to receive, and connect to, an operative member, the operative member being for insertion into a patient's airway in use.

The body portion 20 is a substantially cylindrical body, having a circumference and length similar to that of a user's hand grip, such that the body portion 20 can be easily held in one hand by a user. The body portion 20 has a substantially concave rear edge, such that the head portion 40 and the lower end of the body portion 20 extend further outwards at the rear of the endoscope 10, further aiding a user's grip of the endoscope 10.

The head portion 40 is also a substantially cylindrical body of a similar circumference to the body portion 20. The arm portion 30 extends substantially perpendicularly to the body portion 20, such that the body portion 20 and the arm portion 30 form a substantially L-shaped endoscope body. The arm portion 30 is a substantially cuboidal body, with rounded edges, that is substantially less in diameter than the body portion 20 and the head portion 40.

On its front side, the head portion 40 comprises a recess, the recess having the slot 70 therein. Within the slot 70 is an actuator 60 that is vertically moveable from one end of the slot 70 to the other. The slot 70 and the actuator 60 are also positioned on the front side of the head portion 40 such that a user may operate the actuator 60 with their thumb when holding the endoscope 10 and viewing the display screen.

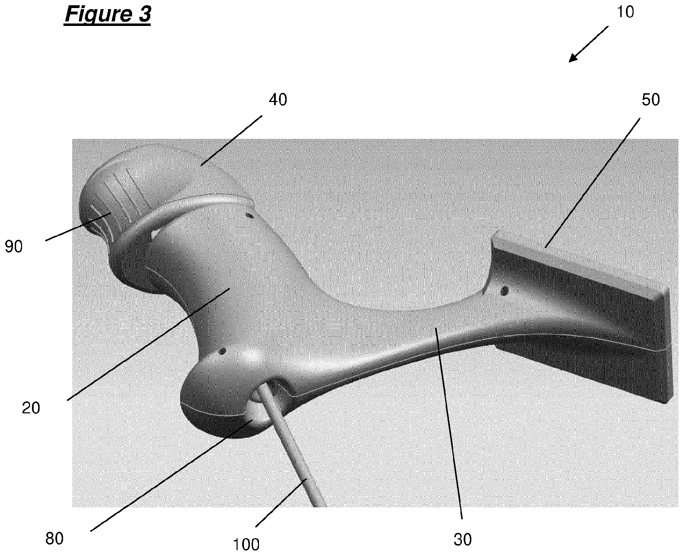

At the bottom end of the body portion 20, ie the surface opposite the head portion 40, is the port 80. The port 80 is substantially circular and is configured to receive, and connect to, a flexible operative member 100 (shown in Figure 3), the operative member 100 being for insertion into a patient in use.

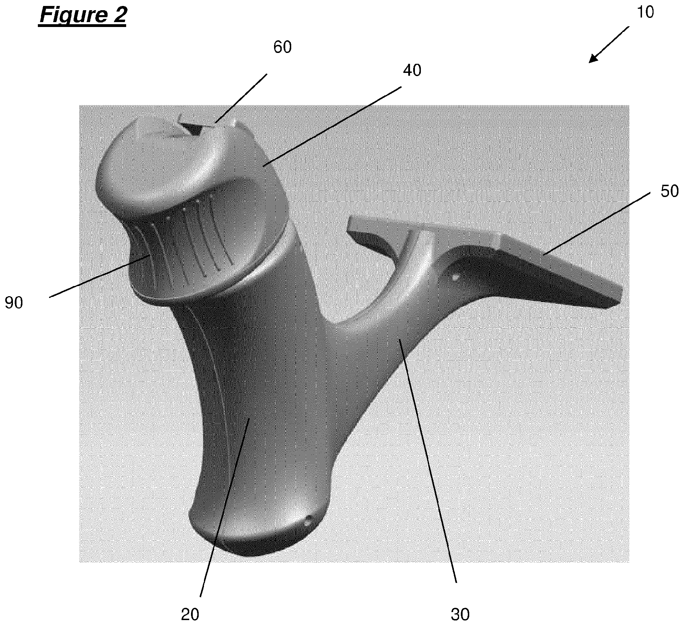

On the opposing side of the head portion 40 to the slot 70 and the actuator 60 is a grip portion 90 (see Figure 2). The grip portion 90 is positioned on the rear side of the head portion 40 such that it may be contacted by a first finger of the user when holding the endoscope 10.

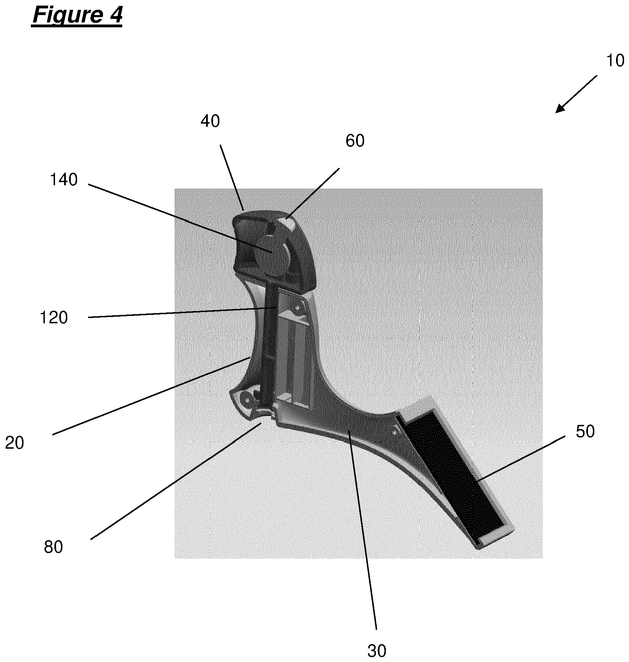

Figure 4 shows a cross-sectional view through the endoscope 10 of Figures 1-3, which illustrates the mechanics by which the head portion 40 and the actuator 60 control movement of an operative member 100 received within port 80.

The head portion 40 is rotatable relative to the body portion 20, and fixedly connected to the operative member 100 via connecting member 120. The head portion 40 and the connecting member 120 may be formed as a unitary component for insertion into a corresponding receiving channel running through the body portion 20. In use, an operative member 100 is inserted through port 80 and fixedly connected to the connecting member 120. Connecting member 120 extends between the port 80 and the head portion 40 through the body portion 20, mechanically connecting the head portion 40 to the operative member 100 received therein.

Rotation of the head portion 40 relative to the body portion 20 therefore causes corresponding rotation of the connecting member 120 and the operative member 100 relative to the body portion 20, ie about the longitudinal axis of the operative member 100. Thus, rotation of the head portion 40 in an anticlockwise direction causes rotation of the operative member 100 in an anticlockwise direction, and rotation of the head portion 40 in a clockwise direction causes rotation of the operative member 100 in a clockwise direction.

The grip portion 90 is particularly useful for this function since it allows a user holding the endoscope to rotate the head portion 40 using only a first finger contacting the grip portion 90.

This arrangement allows a user to rotate the head portion 40, and thus rotate the operative member 100, with just a single finger, whilst keeping the body portion 20 still with the remainder of their hand. This relative movement enables the user to control the operative member 100 without changing the orientation of the display 50 relative to themselves, thus allowing them to maintain a constant view of the display, and thus the patient's trachea, in use.

The actuator 60 comprises a lever moveable within the slot 70 so as to control a movement of the operative member 100, in use. In particular, the actuator is moveable within the slot 70 so as to control the orientation of a distal tip portion of the operative member 100, such that the distal tip portion of the operative member 100 is movable to a range of oblique angles relative to a main body of the operative member 100. In use, the operative member 100 is inserted into the trachea of a patient and the distal tip portion of the operative member 100 can then be moved relative to the main body of the operative member 100. An appropriate mechanism is shown in detail in US 2018/0303317 A1.

The connecting member 120 comprises a channel, through which a pair of threads connect actuator 60 to an operative member 100 received within port 80. The actuator 60 is mechanically connected to a wheel 140, which in turn is connected to the pair of threads at a first end of each thread. The threads are connected to the operative member 100 at a second end of each thread.

As the actuator 60 is moved one way within slot 70, it will rotate the wheel 140 in an anticlockwise direction, thus tightening a first of the threads and bending the distal tip portion such that it is angled relative to the main body of the operative member 100 in a first direction. As the actuator 60 is moved in the opposite direction within slot 70, it will rotate the wheel 140 in the clockwise direction, thus tightening the second of the threads and bending the distal tip portion such that it is angled relative to the main body of the operative member 100 in a second, opposite direction. Hence, when the actuator 60 is positioned centrally within the slot 70, the distal tip portion is aligned with the main body of the operative member 100, rather than angled.

It will be appreciated that alternative known actuation mechanisms may be implemented to control both rotation of the operative member 100 and articulation of the distal tip portion of the operative member 100.

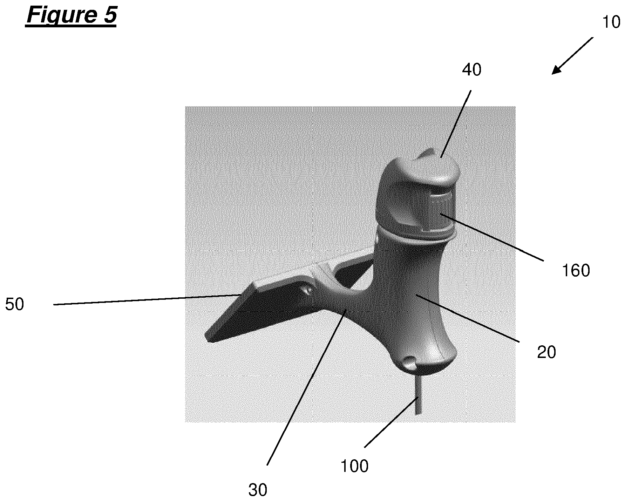

One such alternative for controlling rotation of the operative member 100 is illustrated in Figure 5. In this alternative embodiment, the endoscope 10 comprises a wheel 160 positioned within a slot on a rear face of the head portion 40. In this embodiment, the head portion 40 is fixed relative to the body portion 20, and instead the wheel 160 controls rotational movement of the operative member 100. The wheel 160 is rotatable within the head portion 40, and is therefore rotatable relative to both the head portion 40 and the body portion 20.

Similar to the embodiment illustrated in Figure 4, the operative member 100 is inserted through port 80 and fixedly received within a channel, eg with a snap fit. The channel extends between the port 80 and the wheel 160 through the body portion 20 and the head portion 40, mechanically connecting the wheel 160 to the operative member 100 received therein. Rotation of the wheel 160 within the head portion 40 therefore causes corresponding rotation of the channel 120 and the operative member 100 relative to the body portion 20, ie about the longitudinal axis of the operative member 100.

Thus, rotation of the wheel 160 in an anticlockwise direction causes rotation of the operative member 100 in an anticlockwise direction, and rotation of the wheel 160 in a clockwise direction causes rotation of the operative member 100 in a clockwise direction.

Similar to the embodiment described in relation to Figure 4, this arrangement allows a user to rotate the wheel 160 within the head portion 40, and thus rotate the operative member 100, without changing the orientation of the display 50, allowing them to maintain a constant view of the display, and thus the patient's trachea, in use.

Other alternative actuation mechanisms for controlling rotation of the operative member and/or articulation of the distal tip portion of the operative member may include, for example, the above-described wheel mechanism 160 housed within the body portion 20, or an additional actuating arrangement similar to the actuator 60 and slot 70 described above for use in rotation of the operative member..

The operative member 100 comprises a light source such as an LED at its distal tip, ie the end of the operative member 100 that enters the trachea of a patient first during use. The light source is used to illuminate an interior surface of the patient, from which the images transmitted to the display device are obtained. The operative member 100 comprises one or more light guides, eg optical fibres, that extend from the body portion 20 to the distal end of the operative member 100, through which the images are relayed to the display device.

The distal tip portion of the operative member 100 comprises an image sensor for converting the images into electronic format, which is disposed at the distal end of the operative member 100. The images may be in the form of single images or video. These images are relayed to, and displayed by, the display screen 50. The images may be transmitted in electronic format to the display screen 50 by a wired connection though the operative member 100 and the body and arm portions 20,30 to the display screen 50.

The distal tip of the operative member 100 is formable, eg by bending. In particular, the distal tip of the operative member 100 is malleable, such that it may be bent relative to the main portion of the operative member 100 as described above.

The display portion 50, and the display screen therein, are substantially square shaped. The display portion 50 is tilted relative to the vertical axis of the body portion 20 such that the display screen tilts slightly upwards relative to the vertical axis of the body portion 20. This makes viewing of the display screen easier when the endoscope 10 is being used by an operator.

In use, the operative member 100 is inserted into an endotracheal tube to image the trachea. The endoscope 10 and the endotracheal tube are then introduced into the trachea, such that a distal end of the endotracheal tube is located within the trachea, and a proximal end of the endotracheal tube projects from the patient's mouth. As the operative member 100 transmits images to the display screen 50, a user can see when the endoscope 10 passes through the patient's trachea, and since the endotracheal tube is pre-loaded onto the endoscope 10, will thus know when the endotracheal tube passes through the patient's trachea. The location of the distal tip portion of the operative member 100 is controlled by the user using the actuator 60 and the rotatable head portion 40, such that the endoscope 10 and the endotracheal tube are guided appropriately, and the display screen 50 provides images of the body during insertion to facilitate this guiding of the endotracheal tube. Once the distal end of the endotracheal tube is appropriately positioned within the trachea, the operative member 100 is withdrawn, along with the laryngoscope.

Alternatively, where the endotracheal tube is not pre-loaded onto the endoscope 10, the endoscope 10 may be positioned just outside of the trachea so as to image the trachea as an endotracheal tube is inserted.

## Claims

1. An endoscope (10) for use in intubation, the endoscope (10) comprising a housing, a display device having a display screen (50), and an operative member (100) extending from the housing, the operative member (100) being configured to be inserted into a patient's airway and to transmit images to the display screen (50), the housing comprising an actuator coupled to the operative member (100) and a retainer coupled to the display screen (50), the housing having a handle portion comprising at least the actuator, and the actuator being single-handedly rotatable by a user relative to the retainer, such that the operative member is rotatable relative to the display screen, **characterized in that** the actuator and the retainer are rotatable relative to each other about a longitudinal axis of the operative member (100) and/or a longitudinal axis of the handle portion.

2. An endoscope (10) according to Claim 1, wherein the actuator and the retainer are arranged such that they are engageable by first and second parts of an operator's body, the first and second parts of the operator's body being capable of movement relative to each other.

3. An endoscope (10) according to Claim 2, wherein the actuator is arranged to be engageable by at least one finger of the operator, and the retainer is arranged to be engageable by at least one different finger of the operator, such that the operator may effect rotation of the actuator and the retainer relative to each other through relative movement of their fingers.

4. An endoscope (10) according to any preceding claim, wherein the handle portion comprises the actuator and the retainer.

5. An endoscope (10) according to any preceding claim, wherein the actuator and the retainer are rotatable relative to each other about a longitudinal axis of the operative member (100).

6. An endoscope (10) according to any preceding claim, wherein the actuator and the retainer are rotatable relative to each other about a longitudinal axis of the handle portion.

7. An endoscope (10) according to any preceding claim, wherein the operative member (100) extends from the housing substantially parallel to the longitudinal axis of the handle portion.

8. An endoscope (10) according to any preceding claim, wherein the actuator (40, 160) comprises an actuator member that is pivotable or rotatable about a longitudinal axis of the operative member (100) and/or the handle portion.

9. An endoscope (10) according to any preceding claim, wherein the actuator comprises a single actuator for controlling rotation of the operative member (100) relative to the handle portion and for controlling movement of the distal tip portion of the operative member (100).

10. An endoscope (10) according to any preceding claim, wherein the display screen (50) is spatially separated from the handle portion and a user's wrist is positionable between the display screen (50) and the end of the housing from which the operative member (100) extends.

11. An endoscope (10) according to any preceding claim, wherein the housing includes an arm portion (30) that extends between the handle portion and the display screen device (50).

12. An endoscope (10) according to Claim 11, wherein the arm portion (30) extends from the end of the handle portion from which the operative member (100) extends and/or wherein the arm portion (30) is arranged to rest against the inside of the operator's wrist.

13. An endoscope (10) according to any preceding claim, wherein the display device is accommodated within a display screen portion of the housing that is formed integrally with at least the handle portion of the housing, and/ or wherein the housing of the endoscope (10) comprises at least one housing component that defines a portion of the handle housing portion and the display screen housing portion.

14. A method of manufacturing an endoscope (10) as claimed in any preceding claim, the method comprising the steps of:
(i) providing a housing, a display device having a display screen (50), and an operative member (100) extending from the housing, the operative member (100) being configured to be inserted into a patient's airway and to transmit images to the display screen (50), and the housing comprising an actuator coupled to the operative member and a retainer coupled to the display screen (50), and
(ii) arranging the housing to have a handle portion comprising at least the actuator, and the actuator to be single-handedly rotatable by a user relative to the retainer, such that the operative member (100) is rotatable relative to the display screen, **characterized in that** the actuator and the retainer are rotatable relative to each other about a longitudinal axis of the operative member (100) and/or a longitudinal axis of the handle portion.

## Patentansprüche

1. Endoskop (10) zur Verwendung bei der Intubation, wobei das Endoskop (10) ein Gehäuse, eine Anzeigevorrichtung mit einem Anzeigebildschirm (50) und ein Funktionselement (100), das sich von dem Gehäuse erstreckt, umfasst, wobei das Funktionselement (100) dazu konfiguriert ist, in den Atemweg eines Patienten eingeführt zu werden und Bilder auf den Anzeigebildschirm (50) zu übertragen, wobei das Gehäuse einen Aktuator, der mit dem Funktionselement (100) gekoppelt ist, und eine Halterung, die mit dem Anzeigebildschirm (50) gekoppelt ist, umfasst, wobei das Gehäuse einen Griffabschnitt aufweist, der mindestens den Aktuator umfasst, der Aktuator einhändig durch einen Benutzer relativ zu der Halterung drehbar ist, so dass das Funktionselement relativ zu dem Anzeigebildschirm drehbar ist, **dadurch gekennzeichnet, dass** der Aktuator und die Halterung relativ zueinander um eine Längsachse des Funktionselements (100) und/oder eine Längsachse des Griffabschnitts drehbar sind.

2. Endoskop (10) nach Anspruch 1, wobei der Aktuator und die Halterung so angeordnet sind, dass sie mit einem ersten und einem zweiten Teil des Körpers einer Bedienungsperson in Eingriff bringbar sind, wobei der erste und der zweite Teil des Körpers der Bedienungsperson zu einer Bewegung relativ zueinander fähig sind.

3. Endoskop (10) nach Anspruch 2, wobei der Aktuator so angeordnet ist, dass er mit mindestens einem Finger der Bedienungsperson in Eingriff bringbar ist, und die Halterung so angeordnet ist, dass sie mit mindestens einem anderen Finger der Bedienungsperson in Eingriff bringbar ist, so dass die Bedienungsperson eine Drehung des Aktuators und der Halterung relativ zueinander durch eine relative Bewegung ihrer Finger bewirken kann.

4. Endoskop (10) nach einem vorhergehenden Anspruch, wobei der Griffabschnitt den Aktuator und die Halterung umfasst.

5. Endoskop (10) nach einem vorhergehenden Anspruch, wobei der Aktuator und die Halterung relativ zueinander um eine Längsachse des Funktionselements (100) drehbar sind.

6. Endoskop (10) nach einem vorhergehenden Anspruch, wobei der Aktuator und die Halterung relativ zueinander um eine Längsachse des Griffabschnitts drehbar sind.

7. Endoskop (10) nach einem vorhergehenden Anspruch, wobei sich das Funktionselement (100) von dem Gehäuse im Wesentlichen parallel zu der Längsachse des Griffabschnitts erstreckt.

8. Endoskop (10) nach einem vorhergehenden Anspruch, wobei der Aktuator (40, 160) ein Aktuatorelement umfasst, das um eine Längsachse des Funktionselements (100) und/oder des Griffabschnitts schwenkbar oder drehbar ist.

9. Endoskop (10) nach einem vorhergehenden Anspruch, wobei der Aktuator einen einzigen Aktuator zum Steuern einer Drehung des Funktionselements (100) relativ zum Griffabschnitt und zum Steuern einer Bewegung des distalen Spitzenabschnitts des Funktionselements (100) umfasst.

10. Endoskop (10) nach einem vorhergehenden Anspruch, wobei der Anzeigebildschirm (50) räumlich von dem Griffabschnitt getrennt ist und das Handgelenk eines Benutzers zwischen dem Anzeigebildschirm (50) und dem Ende des Gehäuses, von dem aus sich das Funktionselement (100) erstreckt, positionierbar ist.

11. Endoskop (10) nach einem vorhergehenden Anspruch, wobei das Gehäuse einen Armabschnitt (30) enthält, der sich zwischen dem Griffabschnitt und der Anzeigebildschirmvorrichtung (50) erstreckt.

12. Endoskop (10) nach Anspruch 11, wobei sich der Armabschnitt (30) von dem Ende des Griffabschnitts erstreckt, von dem sich das Funktionselement (100) erstreckt, und/oder wobei der Armabschnitt (30) so angeordnet ist, dass er an der Innenseite des Handgelenks der Bedienungsperson anliegt.

13. Endoskop (10) nach einem vorhergehenden Anspruch, wobei die Anzeigevorrichtung innerhalb eines Anzeigebildschirmabschnitts des Gehäuses untergebracht ist, der einstückig mit mindestens dem Griffabschnitt des Gehäuses ausgebildet ist, und/oder wobei das Gehäuse des Endoskops (10) mindestens eine Gehäusekomponente umfasst, die einen Abschnitt des Griffgehäuseabschnitts und des Anzeigebildschirmgehäuseabschnitts definiert.

14. Verfahren zum Herstellen eines Endoskops (10) nach einem vorhergehenden Anspruch, wobei das Verfahren die folgenden Schritte umfasst:
(i) Bereitstellen eines Gehäuses, einer Anzeigevorrichtung mit einem Anzeigebildschirm (50) und eines Funktionselements (100), das sich von dem Gehäuse erstreckt, wobei das Funktionselement (100) dazu konfiguriert ist, in den Atemweg eines Patienten eingeführt zu werden und Bilder auf den Anzeigebildschirm (50) zu übertragen, und wobei das Gehäuse einen Aktuator, der mit dem Funktionselement gekoppelt ist, und eine Halterung, die mit dem Anzeigebildschirm (50) gekoppelt ist, umfasst, und
(ii) Anordnen des Gehäuses, sodass es einen Griffabschnitt aufweist, der mindestens den Aktuator umfasst, des Aktuators, sodass er einhändig durch einen Benutzer relativ zu der Halterung drehbar ist, so dass das Funktionselement (100) relativ zu dem Anzeigebildschirm drehbar ist, **dadurch gekennzeichnet, dass** der Aktuator und die Halterung relativ zueinander um eine Längsachse des Funktionselements (100) und/oder eine Längsachse des Griffabschnitts drehbar sind.

## Revendications

1. Endoscope (10) destiné à être utilisé pour l'intubation, l'endoscope (10) comprenant un boîtier, un dispositif d'affichage comportant un écran d'affichage (50) et un élément fonctionnel (100) s'étendant depuis le boîtier, l'élément fonctionnel (100) étant conçu pour être inséré dans les voies respiratoires d'un patient et pour transmettre des images à l'écran d'affichage (50), le boîtier comprenant un actionneur couplé à l'élément fonctionnel (100) et un dispositif de retenue couplé à l'écran d'affichage (50), le boîtier comportant une partie poignée comprenant au moins l'actionneur, et l'actionneur pouvant être tourné d'une seule main par un utilisateur par rapport au dispositif de retenue, de sorte que l'élément fonctionnel puisse tourner par rapport à l'écran d'affichage, **caractérisé en ce que** l'actionneur et le dispositif de retenue peuvent tourner l'un par rapport à l'autre autour d'un axe longitudinal de l'élément fonctionnel (100) et/ou d'un axe longitudinal de la portion poignée.

2. Endoscope (10) selon la revendication 1, ledit actionneur et ledit dispositif de retenue étant agencés de sorte qu'ils puissent être mis en prise par des première et seconde parties du corps d'un opérateur, les première et seconde parties du corps de l'opérateur pouvant bouger l'une par rapport à l'autre.

3. Endoscope (10) selon la revendication 2, ledit actionneur étant agencé pour pouvoir être engagé par au moins un doigt de l'opérateur, et ledit dispositif de retenue étant agencé pour être engagé par au moins un doigt différent de l'opérateur, de sorte que l'opérateur puisse effectuer la rotation de l'actionneur et du dispositif de retenue l'un par rapport à l'autre par un mouvement relatif de leurs doigts.

4. Endoscope (10) selon l'une quelconque des revendications précédentes, ladite partie poignée comprenant l'actionneur et le dispositif de retenue.

5. Endoscope (10) selon l'une quelconque des revendications précédentes, ledit actionneur et ledit dispositif de retenue pouvant tourner l'un par rapport à l'autre autour d'un axe longitudinal de l'élément fonctionnel (100).

6. Endoscope (10) selon l'une quelconque des revendications précédentes, ledit actionneur et ledit dispositif de retenue pouvant tourner l'un par rapport à l'autre autour d'un axe longitudinal de la partie poignée.

7. Endoscope (10) selon l'une quelconque des revendications précédentes, ledit élément fonctionnel (100) s'étendant depuis le boîtier sensiblement parallèlement à l'axe longitudinal de la partie poignée.

8. Endoscope (10) selon l'une quelconque des revendications précédentes, ledit actionneur (40, 160) comprenant un élément actionneur qui peut pivoter ou tourner autour d'un axe longitudinal de l'élément fonctionnel (100) et/ou de la partie poignée.

9. Endoscope (10) selon l'une quelconque des revendications précédentes, ledit actionneur comprenant un seul actionneur pour contrôler la rotation de l'élément fonctionnel (100) par rapport à la partie poignée et pour contrôler le mouvement de la partie pointe distale de l'élément fonctionnel (100).

10. Endoscope (10) selon l'une quelconque des revendications précédentes, ledit écran d'affichage (50) étant spatialement séparé de la partie poignée et le poignet d'un utilisateur pouvant être positionné entre l'écran d'affichage (50) et l'extrémité du boîtier à partir de laquelle l'élément fonctionnel (100) s'étend.

11. Endoscope (10) selon l'une quelconque des revendications précédentes, ledit boîtier comprenant une partie bras (30) qui s'étend entre la partie poignée et le dispositif à écran d'affichage (50).

12. Endoscope (10) selon la revendication 11, ladite partie bras (30) s'étendant depuis l'extrémité de la partie poignée à partir de laquelle s'étend l'élément fonctionnel (100) et/ou ladite partie bras (30) étant agencée pour reposer contre l'intérieur du poignet de l'opérateur.

13. Endoscope (10) selon l'une quelconque des revendications précédentes, ledit dispositif d'affichage étant logé dans une partie écran d'affichage du boîtier qui est formée d'un seul tenant avec au moins la partie poignée du boîtier, et/ou ledit boîtier de l'endoscope (10) comprenant au moins un composant de boîtier qui définit une partie de la partie de boîtier poignée et de la partie de boîtier écran d'affichage.

14. Procédé de fabrication d'un endoscope (10) selon l'une quelconque des revendications précédentes, le procédé comprenant les étapes de :
(i) fourniture d'un boîtier, d'un dispositif d'affichage comportant un écran d'affichage (50) et d'un élément fonctionnel (100) s'étendant depuis le boîtier, l'élément fonctionnel (100) étant conçu pour être inséré dans les voies respiratoires d'un patient et pour transmettre des images à l'écran d'affichage (50), et le boîtier comprenant un actionneur couplé à l'élément fonctionnel et un dispositif de retenue couplé à l'écran d'affichage (50), et
(ii) agencement du boîtier de manière à avoir une partie poignée comprenant au moins l'actionneur, et l'actionneur pouvant être tourné d'une seule main par un utilisateur par rapport au dispositif de retenue, de sorte que l'élément fonctionnel (100) puisse tourner par rapport à l'écran d'affichage, **caractérisé en ce que** l'actionneur et le dispositif de retenue peuvent tourner l'un par rapport à l'autre autour d'un axe longitudinal de l'élément fonctionnel (100) et/ou d'un axe longitudinal de la portion poignée.
